# EUROPÄISCHE PATENTANMELDUNG

(11) **EP 2 229 926 A1**
(43) Veröffentlichungstag der Anmeldung: **22.09.2010**
(21) Anmeldenummer: 10002555.0
(22) Anmeldetag: 11.03.2010
(51) Int. Cl.: A61H 7/00, A61H 23/02, A46B 13/02, A61C 17/20, A46B 7/04, A46B 5/00

(54) **Ultraschallbehandlungsgerät, insbesondere für kosmetische Behandlungen**

(30) Priorität: 16.03.2009 DE 202009003586 U
(71) Anmelder: EMAG AG, 64546 Mörfelden-Walldorf (DE)
(72) Erfinder: Emekci, Bulent, 64546 Mörfelden-Walldorf (DE)
(74) Vertreter: Graf, Helmut

(57) **Zusammenfassung**

Ultraschallbehandlungsgerät zur Behandlung von Hautpartien (17), insbesondere zur kosmetische Behandlung von Hautpartien mit einem abnehmbar an einem Gerätegehäuse (2) vorgesehenen Behandlungskopf (9), mit wenigstens einen in dem Behandlungskopf untergebrachten elektromechanischen Wandler (13) zur Erzeugung von Ultraschall-Schwingungen oder -Wellen an einem Arbeitsbereich des Behandlungskopfes, sowie mit einer elektrischen Ansteuerschaltung (4) zur Ansteuerung des Wandlers im Gerätegehäuse.

## Beschreibung

Die Erfindung bezieht sich auf ein Ultraschallbehandlungsgerät gemäß Oberbegriff Patentanspruch 1.

Aufgabe der Erfindung ist es, ein Ultraschallbehandlungsgerät aufzuzeigen, welches zur Behandlung von Hautpartien, insbesondere zur kosmetische Behandlung von Hautpartien, d.h. im kosmetischen Bereich für eine schonende Behandlung von Hautpartien, insbesondere der Gesichtshaut verwendbar ist, und dabei u.a. zum Reinigen und/oder zur Massage.

Zur Lösung dieser Aufgabe ist ein Ultraschallbehandlungsgerät entsprechend dem Patentanspruch 1 ausgebildet. Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an einem Ausführungsbeispiel näher erläutert. Es zeigen:

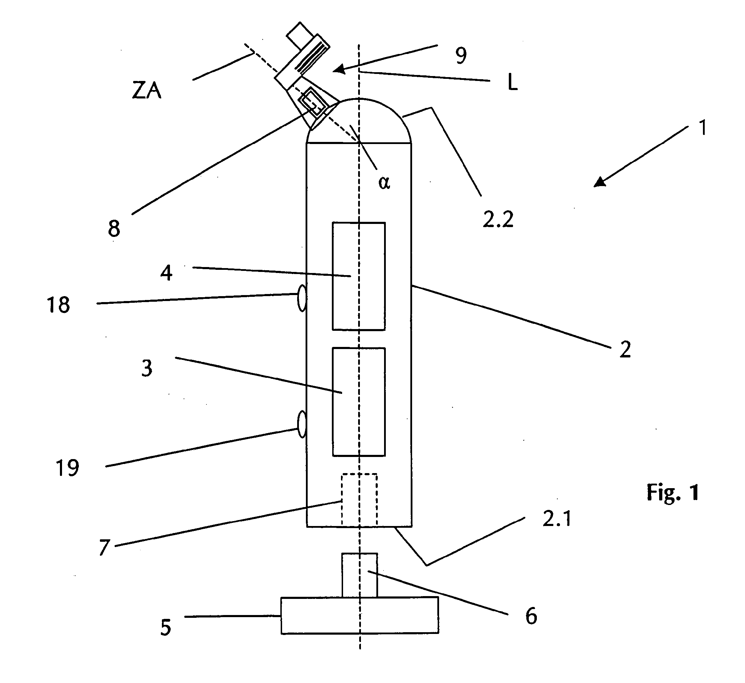

Figur 1 in vereinfachter, schematischer Darstellung ein Ultraschall-Behandlungsgerät gemäß der Erfindung;

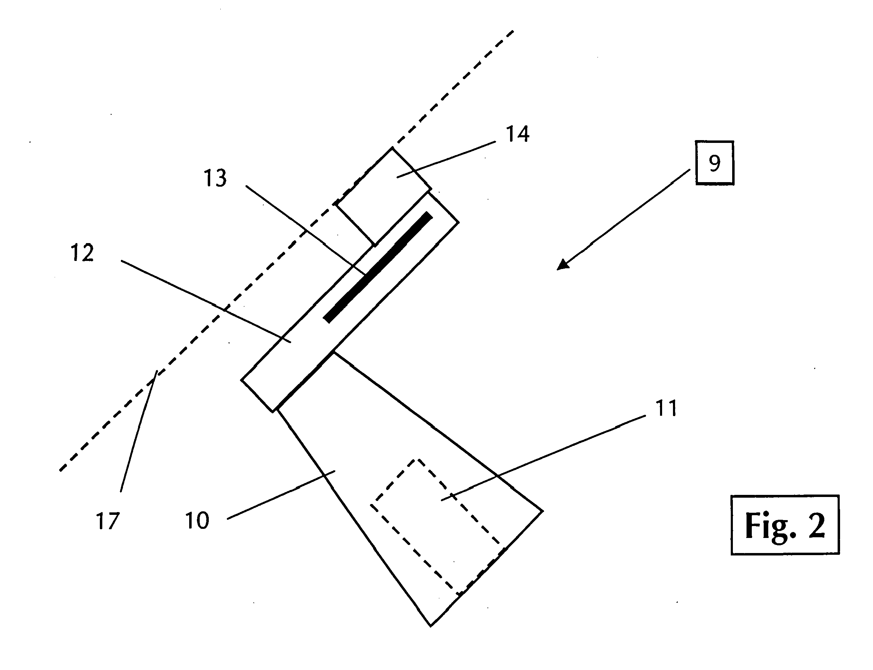

Figur 2 in Einzeldarstellung den Behandlungskopf des Gerätes der Figur 1.

Das in den Figuren allgemein mit 1 bezeichnete Ultraschall-Behandlungsgerät dient zur kosmetischen Behandlung, insbesondere zum Reinigen und/oder zur Massage der Haut, speziell auch der Gesichtshaut, kann aber auch zur Reinigen von Zähnen verwendet werden.

Das Ultraschall-Behandlungsgerät 1 umfasst u.a. einen langgestreckten, im Wesentlichen zylinderförmigen Gerätekörper mit Gerätegehäuse 2, in welchem verschiedene, insbesondere auch elektrische Funktionselemente untergebracht sind, so eine wiederaufladbare Batterie 3 und eine elektrische Schaltung 4, die u.a. den Ladestromkreis für die Batterie 3 sowie auch eine Ansteuerschaltung zur Erzeugung eines elektrischen Wechselspannungssignals mit einer Frequenz im Ultraschallbereich bildet. Über ein äußeres, an das 230 V-Versorgungsnetz anschließbares Ladegerät 5 ist die Batterie 3 aufladbar, und zwar bevorzugt kontaktlos, beispielsweise magnetisch und/oder kapazitiv durch Einführen eines am Ladegerät 5 vorgesehenen Ladezapfens 6 in eine Ausnehmung 7, die bei der für die Figur gewählten Darstellung am unteren Ende 2.1 des Gerätegehäuses vorgesehen ist. An dem oberen, der Ausnehmung 7 entfernt liegenden und bei der dargestellten Ausführungsform abgerundeten Ende 2.2 des Gerätegehäuses 2 ist gegenüber der Längsachse L des Gerätegehäuses 2 radial versetzt ein über die Außenfläche des Gerätegehäuses 2 wegstehender Kupplungszapfen 8 vorgesehen, dessen Zapfenachse ZA mit der Gehäuselängsachse L einen Winkel α < 90° einschließt, der sich zu dem Ende 2.2 des Gerätegehäuses 2 hin öffnet.

Der Kupplungszapfen 8 dient zur abnehmbaren Befestigung eines Arbeits- oder Behandlungskopfes 9, der bei der dargestellten Ausführungsform als Bürstenkopf ausgebildet ist. Der Behandlungskopf 9 besteht im Wesentlichen aus einem Kopfabschnitt 10, der bei der dargestellten Ausführungsform kegelartig ausgeführt ist und an einem Ende eine Kupplungsöffnung 11 aufweist, mit der der Behandlungskopf 9 auf den Kupplungszapfen 8 aufgesetzt werden kann. Die Kupplungsöffnung 11 ist beispielsweise mit einem Innengewinde versehen, welchem ein Außengewinde am Zapfen 8 zugeordnet ist, sodass die Befestigung des Behandlungskopfes 9 am Gerät 1 bzw. am Gerätegehäuse 2 durch Aufschrauben auf den Zapfen 8 erfolgt. Auch andere Möglichkeiten einer Sicherung des Behandlungskopfes 9 am Zapfen 8 sind denkbar, beispielsweise durch Aufschieben des Kopfabschnittes 10 mit seiner Öffnung 11 auf dem Zapfen 8 und durch Verrasten des Kopfabschnittes 10 am Zapfen 8 oder durch Verriegeln des Kopfabschnittes 10 am Zapfen 8 durch eine Art Bajonettverriegelung oder über ein kurzes Gewinde, beispielsweise über ein Gewinde, welches die Verriegelung des Kopfabschnittes 10 am Zapfen 8 und damit die Sicherung des Behandlungskopfes 9 am Gerätegehäuse 2 sowie das Lösen des Behandlungskopfes vom Gerätegehäuse 2 durch ein Drehen des Behandlungskopfes 9 um einen Winkel kleiner als 360° ermöglicht.

Der Kopfabschnitt 10 ist einstückig mit einem weiteren Kopfabschnitt 12 ausgeführt, der an dem der Öffnung 11 entfernt liegenden Ende des Kopfabschnittes 10 von diesem radial wegsteht. In dem Kopfabschnitt 12 ist ein elektromechanischer Wandler vorgesehen, der bei am Gerätegehäuse 2 befestigtem Behandlungskopf 9 das von der Schaltung 4 gelieferte elektrische Signal in ein mechanisches Ultraschall-Signal umwandelt, beispielsweise in eine Ultraschall-Schwingung oder in eine UltraschallWelle.

Der Wandler 13, der beispielsweise ein Piezo-Wandler ist, ist hierfür über elektrische Ansteuerleitungen und elektrische Kontakte im Bereich der von dem Vorsprung 8 und der Öffnung 9 gebildeten mechanischen und elektrischen Kupplung mit der Schaltung 4 verbunden. Der Wandler 13 befindet sich bei der dargestellten Ausführungsform unmittelbar unterhalb einer Bürstenanordnung 14, die von einer Vielzahl von Borsten gebildet und an dem freien Ende des Kopfabschnittes 12 vorgesehen ist, und zwar an derjenigen Seite des Kopfabschnittes 12, die bei mit dem Gerätegehäuse 12 verbundenem Arbeitskopf 9 dem Gerätegehäuse 2 abgewandt ist.

Durch die gekröpfte Ausbildung des Arbeitskopfes 9 mit den beiden Kopfsabschnitten 10 und 12 ist die Unterbringung des Wandlers 13 im Behandlungskopf 9 möglich. Am Gerätegehäuse 2 sind weiterhin wenigstens ein Betätigungselement 15 für das manuelle Ein- und Ausschalten des Gerätes 1 sowie zumindest eine Kontroll-Leuchte 16, beispielsweise zur Anzeige des jeweiligen Betriebszustandes und/oder des Ladezustandes der Batterie 3 vorgesehen.

Im Verwendungsfall wird der Arbeitskopf 9 mit seinem Arbeitsabschnitt, d.h. bei der dargestellten Ausführungsform mit dem Bürstenabschnitt 14 gegen den zu behandelnden Hautbereich 17 zur Anlage gebracht, sodass die von dem Wandler 13 erzeugten Ultraschall-Schwingungen oder -Wellen über die Borsten der Bürstenanordnung 14 auf die zu behandelnde Hautpartie 17 übertragen werden. Durch die beschriebene geköpfte Formgebung des Arbeitskopfes 9 und durch die beschriebene Anordnung des Kupplungszapfens 8 und durch die dadurch sich ergebende gegenüber der Längsachse L geneigte Arbeitsebene E des Behandlungskopfes 8 ist ein bequemes und unbehindertes Führen des Bürstenabschnitts 14 entlang der zu behandelden Hautpartie 17 möglich.

Die Behandlung erfolgt bei der dargestellten Ausführungsform ausschließlich mit den vom Wandler 13 erzeugten Ultraschall-Schwingungen und/oder -Wellen. Ein weiterer mechanischer Antrieb ist nicht vorgesehen. Nach Abschluss der Behandlung wird das Gerät 1 an die Ladestation 5 zum Nachladen der Batterie 3 durch Einführen des Ladezapfens 6 in die Ausnehmung 7 angedockt.

Die Erfindung wurde voranstehend an einem Ausführungsbeispiel beschrieben. Es versteht sich, dass Änderungen sowie Abwandlungen möglich sind, ohne dass dadurch der der Erfindung zugrundeliegende Erfindungsgedanke verlassen wird.

### Bezugszeichenliste

- 1: Ultraschallbehandlungsgerät
- 2: Gerätekörper
- 2.1, 2.2: Ende des Gerätekörpers
- 3: wiederaufladbare Batterie bzw. Akku
- 4: elektronische Schaltung
- 6: Ladezapfen
- 7: Ladeöffnung
- 8: Kupplungszapfen
- 9: Arbeitskopf
- 10: Kopfabschnitt
- 11: Kupplungsöffnung
- 12: Kopfabschnitt
- 13: elektromechanischer Wandler
- 14: Bürstenanordnung
- 15: manuell betätigbares Betätigungselemente
- 16: Kontrollleuchte
- 17: Hautpartie
- E: Arbeitseben
- L: Längsachse des Gerätegehäuses 2
- ZA: Zapfenachse
- α: Winkel

## Patentansprüche

1. Ultraschallbehandlungsgerät, insbesondere für kosmetische Behandlung von Hautpartien (17), **gekennzeichnet durch** einen abnehmbar an einem Gerätekörper oder -gehäuse (2) vorgesehenen Behandlungskopf (9) mit wenigstens einen in dem Behandlungskopf untergebrachten elektromechanischen Wandler (13) zur Erzeugung von Ultraschall-Schwingungen oder -Wellen an einem Arbeitsbereich (14) des Behandlungskopfes, sowie **durch** eine elektrische Ansteuerschaltung (4) zur Ansteuerung des Wandlers (13) im Gerätegehäuse (2).

2. Gerät nach Anspruch 1, **dadurch gekennzeichnet, dass** im Gerätegehäuse (2) eine Spannungsversorgung vorzugsweise in Form einer wiederaufladbaren Batterie (3) vorgesehen ist.

3. Gerät nach Anspruch 1 oder 2, **dadurch gekennzeichnet, dass** der Behandlungskopf (9) abnehmbar und/oder austauschbar über eine mechanische und elektrische Kupplung am Gerätegehäuse (2) vorgesehen ist.

4. Gerät nach einem der vorhergehenden Ansprüche, **dadurch gekennzeichnet, dass** der Arbeitsbereich des Behandlungskopfes (9) von einem Bürstenabschnitt (14) bestehend aus mehreren Borsten gebildet ist.

5. Gerät nach einem der vorhergehenden Ansprüche, **dadurch gekennzeichnet, dass** der Behandlungskopf (9) gekröpft mit einem ersten mit dem Gerätegehäuse (2) verbundenen oder verbindbaren Kopfabschnitt (10) und mit einem zweiten, von dem ersten Kopfabschnitt radial oder im Wesentlichen radial wegstehenden und den Arbeitsbereich (14) des Behandlungskopfes (9) aufweisenden Kopfabschnitt (12) ausgebildet ist, und dass im zweiten Kopfabschnitt (12) der Wandler (13) untergebracht ist.

6. Gerät nach einem der vorhergehenden Ansprüche, **dadurch gekennzeichnet, dass** sich der Wandler (13) im Behandlungskopf (9) nach außen hin verschlossen unmittelbar unter dem Arbeitsbereich (14) befindet.

7. Gerät nach einem der vorhergehenden Ansprüche, **dadurch gekennzeichnet, dass** der mit dem Gerätekörper (2) verbundene Behandlungskopf (9) zumindest mit seinem ersten Kopfabschnitt (10) gegenüber einer Längsachse (L) des Gerätegehäuses (2) radial versetzt und/oder derart geneigt vorgesehen ist, dass die Achse (ZA) des ersten Kopfabschnittes (10) mit der Gerätegehäuselängsachse (L) einen Winkel (α) kleiner als 90° einschließt.

8. Gerät nach einem der vorhergehenden Ansprüche, **dadurch gekennzeichnet, dass** die Arbeitsebene des Behandlungsabschnitts (14) gegenüber einer Längserstreckung (L) des Gerätegehäuses (2) geneigt ist.

9. Gerät nach einem der vorhergehenden Ansprüche, **gekennzeichnet durch** eine mechanische und elektrische Kupplung zum lösbaren Verbinden des Behandlungskopfes (9) mit dem Gerätegehäuse (2), wobei die Kupplung bevorzugt von einem Kupplungszapfen (8) am Gerätegehäuse (2) und einer passenden Kupplungsöffnung (11) am Behandlungskopf (9) gebildet ist und der Kupplungszapfen (8) vorzugsweise gegenüber einer Längserstreckung (L) des Gerätegehäuses (2) radial versetzt ist und/oder eine Zapfenachse (ZA) des Kupplungszapfens (8) mit der Längserstreckung (L) einen Winkel (α) kleiner als 90° einschließt.

10. Gerät nach einem der vorhergehenden Ansprüche, **dadurch gekennzeichnet, dass** das Gerätegehäuse (2) und/oder der Behandlungskopf bzw. dessen Kopfabschnitt (10, 12) als Formteile aus Kunststoff gefertigt sind.

## Geänderte Patentansprüche

### Geänderte Patentansprüche gemäss Regel 137(2) EPÜ.

**1.** Ultraschallbehandlungsgerät, für kosmetische Behandlung von Hautpartien (17) oder zum Reinigen von Zähnen, mit einem Gerätegehäuse, einem abnehmbar an dem Gerätegehäuse (2) vorgesehenen Behandlungskopf (9), mit wenigstens einem in dem Behandlungskopf untergebrachten elektromechanischen Wandler (13) zur Erzeugung von UltraschallSchwingungen oder -Wellen an einem Arbeitsbereich (14) des Behandlungskopfes, sowie durch eine elektrische Ansteuerschaltung (4) zur Ansteuerung des Wandlers (13) im Gerätegehäuse (2), **dadurch gekennzeichnet, dass** der Behandlungskopf (9) gekröpft mit einem ersten mit dem Gerätegehäuse (2) verbundenen oder verbindbaren Kopfabschnitt (10) und mit einem zweiten, von dem ersten Kopfabschnitt (10) radial oder im Wesentlichen radial wegstehenden und den Arbeitsbereich (14) des Behandlungskopfes (9) aufweisenden Kopfabschnitt (12) ausgebildet ist und dass der mit dem Gerätekörper (2) verbundene Behandlungskopf (9) zumindest mit seinem ersten Kopfabschnitt (10) gegenüber einer Längsachse (L) des Gerätegehäuses (2) radial versetzt und/oder derart geneigt vorgesehen ist, dass die Achse (ZA) des ersten Kopfabschnittes (10) mit der Gerätegehäuselängsachse (L) einen Winkel (α) kleiner als 90° einschließt.

**2.** Gerät nach Anspruch 1, **dadurch gekennzeichnet, dass** im Gerätegehäuse (2) eine Spannungsversorgung vorzugsweise in Form einer wiederaufladbaren Batterie (3) vorgesehen ist.

**3.** Gerät nach Anspruch 1 oder 2, **dadurch gekennzeichnet, dass** der Behandlungskopf (9) abnehmbar und/oder austauschbar über eine mechanische und elektrische Kupplung am Gerätegehäuse (2) vorgesehen ist.

**4.** Gerät nach einem der vorhergehenden Ansprüche, **dadurch gekennzeichnet, dass** der Arbeitsbereich des Behandlungskopfes (9) von einem Bürstenabschnitt (14) bestehend aus mehreren Borsten gebildet ist.

**5.** Gerät nach einem der vorhergehenden Ansprüche, **dadurch gekennzeichnet, dass** im zweiten Kopfabschnitt (12) der Wandler (13) untergebracht ist.

**6.** Gerät nach einem der vorhergehenden Ansprüche, **dadurch gekennzeichnet, dass** sich der Wandler (13) im Behandlungskopf (9) nach außen hin verschlossen unmittelbar unter dem Arbeitsbereich (14) befindet.

**7.** Gerät nach einem der vorhergehenden Ansprüche, **dadurch gekennzeichnet, dass** die Arbeitsebene des Behandlungsabschnitts (14) gegenüber einer Längserstreckung (L) des Gerätegehäuses (2) geneigt ist.

**8.** Gerät nach einem der vorhergehenden Ansprüche, **gekennzeichnet durch** eine mechanische und elektrische Kupplung zum lösbaren Verbinden des Behandlungskopfes (9) mit dem Gerätegehäuse (2), wobei die Kupplung bevorzugt von einem Kupplungszapfen (8) am Gerätegehäuse (2) und einer passenden Kupplungsöffnung (11) am Behandlungskopf (9) gebildet ist und der Kupplungszapfen (8) vorzugsweise gegenüber einer Längserstreckung (L) des Gerätegehäuses (2) radial versetzt ist und/oder eine Zapfenachse (ZA) des Kupplungszapfens (8) mit der Längserstreckung (L) einen Winkel (α) kleiner als 90° einschließt.

**9.** Gerät nach einem der vorhergehenden Ansprüche, **dadurch gekennzeichnet, dass** das Gerätegehäuse (2) und/oder der Behandlungskopf bzw. dessen Kopfabschnitt (10, 12) als Formteile aus Kunststoff gefertigt sind.
